# EUROPÄISCHE PATENTSCHRIFT

(11) **EP 0 809 211 B1**
(45) Veröffentlichungstag und Bekanntmachung des Hinweises auf die Patenterteilung: **11.09.2002**
(21) Anmeldenummer: 97201434.4
(22) Anmeldetag: 13.05.1997
(51) Int. Cl.: G06T 11/00

(54) **Röntgenaufnahme-Verfahren**
Method for radiography
Procédé radiographique

(30) Priorität: 21.05.1996 DE 19620371
(43) Veröffentlichungstag der Anmeldung: 26.11.1997
(73) Patentinhaber: Philips Corporate Intellectual Property GmbH, 52064 Aachen (DE); Koninklijke Philips Electronics N.V., 5621 BA Eindhoven (NL)
(72) Erfinder: Klotz, Erhard Paul Artur, 22335 Hamburg (DE); Koppe, Reiner Heinrich, Dr., 22335 Hamburg (DE); Op de Beek, John, 22335 Hamburg (DE); Aerts, Hans, Dr., 22335 Hamburg (DE)
(74) Vertreter: Hartmann, Heinrich, Dipl.-Ing.

(56) Entgegenhaltungen:
- DE-A- 4 304 571
- US-A- 4 504 858
- US-A- 4 791 934
- US-A- 5 307 264
- FELDMAR J ET AL: "3D-2D PROJECTIVE REGISTRATION OF FREE-FORM CURVES AND SURFACES" PROCEEDINGS OF THE INTERNATIONAL CONFERENCE ON COMPUTER VISION,US,LOS ALAMITOS, IEEE COMP. SOC. PRESS, Bd. CONF. 5, Seite 549-556 XP000557411 ISBN: 0-7803-2925-2

## Beschreibung

Die Erfindung betrifft ein Röntgenaufnahme-Verfahren, bei dem mit einer ersten bildgebenden Einrichtung eine Serie von zweidimensionalen Röntgenaufnahmen angefertigt und digital gespeichert wird, bei denen das Untersuchungsobjekt aus unterschiedlichen Perspektiven auf einen Röntgenbildaufnehmer projiziert wird. Außerdem bezieht sich die Erfindung auf eine Anordnung zur Durchführung des Verfahrens.

Ein solches Verfahren und eine solche Anordnung sind bekannt aus einem Aufsatz von Koppe et al in Proceedings CAR - 95 Berlin, 1995 pp. 101-107 - im folgenden mit D1 bezeichnet. Bevorzugt wird dieses Verfahren zur Darstellung des Gefäßsystems verwendet, in das zuvor Kontrastmittel injiziert wurde. Grundsätzlich wären auch dreidimensionale Darstellungen des Gefäßsystems möglich, z.B. mit MR- oder CT-Aufnahmen. Doch ist es damit noch nicht möglich, das Gefäßsystem mit so hoher räumlicher Auflösung zu rekonstruieren, wie es bei verschiedenen medizinischen Untersuchungen erforderlich ist. Bei dem eingangs beschriebenen Verfahren ergibt sich demgegenüber eine hohe räumliche Auflösung, und es entsteht ein quasi dreidimensionaler räumlicher Eindruck, wenn die Röntgenaufnahmen in schneller Folge nacheinander wiedergegeben werden. Nachteilig dabei ist aber, daß die Röntgenaufnahmen nur das Gefäßsystem zeigen, während das Gewebe in der Umgebung nicht dargestellt wird. Bei stereotaktischen Untersuchungen ist es aber erforderlich, die relative Lage einer für die Untersuchung relevanten Struktur, z.B. eine Tumors, in bezug auf das Gefäßsystem bestimmen zu können.

Zu diesem Zweck ist es aus einer weiteren Veröffenlichung (D2) von Kelly et al in "Neurosurgery", Vol. 14, Nr. 2, 1984 bekannt, einerseits mittels einer Röntgen-Angiographie-Einrichtung Stereobildpaare zu erstellen, die das Gefäßsystem von vorne bzw. von der Seite zeigen und andererseits von der gleichen Region des Untersuchungsobjektes mittels eines Computertomographen ein dreidimensionales Bild zu erstellen. Um die Befunde in den zweidimensionalen Röntgenaufnahmen einerseits und dem dreidimensionalen CT-Bild andererseits einander zuordnen zu können, wird dabei ein Referenzrahmen - gegebenenfalls in Verbindung mit Referenzmarkern verwendet - der bzw. die auf den Röntgenaufnahmen bzw. dem CT-Bild abgebildet werden, so daß die Lage der zu untersuchenden anatomischen Strukturen in bezug auf den Referenzrahmen bzw. die Referenzmarker genau bestimmt und Befunde aus den verschiedenen Abbildungen miteinander korreliert werden können.

Bei dem bekannten Verfahren gibt der Untersucher in dem dreidimensionalen CT-Bild einen Punkt vor, in dem eine Biopsie durchgeführt werden soll, und ein Computer berechnet daraus die mechanischen Einstellungen eines stereotaktischen Rahmens, mit denen der Biopsiepunkt in den Fokuspunkt des stereotaktischen Rahmens gerückt werden kann. Die horizontalen und vertikalen Winkeleinstellungen, unter denen eine Biopsienadel eingeführt wird, werden mit Hilfe der angiographischen Röntgenaufnahmen bestimmt, bei denen die Gefäßpunkte, die dicht am Pfad der Biopsienadel liegen, digitalisiert und in den Computer eingegeben werden, der daraus die geeigneten Winkel berechnet und anzeigt.

Nachteilig bei dieser Prozedur ist, daß der Untersucher sich auf die Berechnungen des Computers verlassen muß und keinen direkten, dreidimensionalen Eindruck der Lage des Biopsiepfades in bezug auf das Gefäßsystem vermittelt bekommt.

Aufgabe der vorliegenden Erfindung ist es, ein Verfahren der eingangs genannten Art so auszugestalten, daß der Benutzer einen verbesserten, quasi dreidimensionalen Eindruck von der Lage der für die Diagnose relevanten Struktur, z.B. eines Tumors, in bezug auf die in den Röntgenaufnahmen dargestellte Anatomie (z.B. das Gefäßsystem) erhält. Für eine Biopsie beispielsweise kann er dann selbst den optimalen Biopsiepfad ermitteln oder einen automatisch vorgegebenen Biopsiepfad beurteilen.

Diese Aufgabe wird erfindungsgemäß gelöst durch die folgenden Verfahrensschritte
a) Erstellung eines dreidimensionalen Bildes von dem gleichen Untersuchungsobjekt mit einer zweiten bildgebenden Einrichtung
b) Extraktion einer relevanten Struktur des Untersuchungsobjektes aus dem dreidimensionalen Bild
c) Für jedes Bild der Serie von zweidimensionalen Röntgenaufnahmen Berechnung eines synthetischen, zweidimensionalen Projektionsbildes der extrahierten Struktur, wobei die Struktur mit den gleichen geometrischen Parametern projiziert wird wie sie für die reale Struktur bei der Erzeugung der einzelnen Röntgenaufnahmen galten
d) Erzeugung von Überlagerungsbildern durch Überlagerung der synthetischen Projektionsbilder und der mit den gleichen geometrischen Parametern erstellten Röntgenaufnahmen
e) Wiedergabe der Folge der Überlagerungsbilder, so daß ein Benutzer einen quasi dreidimensionalen Eindruck von der Lage der relevanten Struktur in Bezug auf das in der Serie von zweidimensionalen Röntgenaufnahmen abgebildete Untersuchungsobjekt erhält.

Bei der Erfindung wird also für jede Röntgenaufnahme ein synthetisches, zweidimensionales Projektionsbild berechnet, das die aus dem dreidimensionalen Bild einer zweiten bildgebenden Einrichtung (die ein Computertomograph sein kann) extrahierte Struktur darstellt, und zwar mit den gleichen geometrischen Parametern, mit denen diese Struktur und z.B. das Gefäßsystem bei der Erzeugung der Röntgenaufnahmen projiziert wird. In den Röntgenaufnahmen ist die Struktur wegen ihres geringen Kontrastes nicht sichtbar; jedoch kann in dem berechneten synthetischen Projektionsbild der Kontrast, mit dem die Struktur dargestellt wird, beliebig vorgegeben werden (es ist auch eine farbige Darstellung möglich). Die Röntgenaufnahme und das synthetische Projektionsbild, die zueinander korrespondieren, werden zu einem Überlagerungsbild kombiniert, und diese Überlagerungsbilder werden als Folge nacheinander wiedergeben. Der Untersucher bekommt dadurch einen quasi dreidimensionalen Eindruck des Untersuchungsbereichs, wobei er die relative Lage der extrahierten Struktur in bezug auf die in den Röntgenaufnahmen dargestellte Anatomie, z.B. das Gefäßsystem, erkennen kann.

Eine bevorzugte Weiterbildung der Erfindung sieht vor, daß als zweite bildgebende Einrichtung ein Röntgen-Computertomograph verwendet wird, wobei zur Erstellung eines dreidimensionalen Bildes von dem Untersuchungsobjekt eine Anzahl von Tomogrammen von parallelen Schichten erstellt wird. Grundsätzlich ist es aber auch möglich, für die Erstellung eines dreidimensionalen Bildes eine andere Modalität zu benutzen, z.B. ein MR-Gerät oder ein Ultraschallgerät.

In weiterer Ausgestaltung der Erfindung ist vorgesehen, daß zur Erzeugung einer Folge von Stereo-Bildpaaren zwei Bild-Folgen wiedergegeben werden, die beide aus der einen Folge der Überlagerungsbilder abgeleitet werden, wobei die beiden Bildfolgen um einige Überlagerungsbilder gegeneinander versetzt sind. Bei geeigneter Wiedergabe erhält der Benutzer dann - für jedes einzelne Überlagerungsbild - einen stereoskopischen Bildeindruck, obwohl nur eine einzige Bildfolge existiert. Wenn die beiden Überlagerungsbilder, die zusammen ein Stereobildpaar darstellen, geeignet aus der Folge ausgewählt werden (z.B. so, daß sie das Objekt aus unter 6° gegeneinander versetzten Perspektiven darstellen), dann entsteht ein stereoskopischer Eindruck, obwohl die beiden Bildfolgen, aus denen die Stereobildpaare abgeleitet werden, nicht voneinander unabhängig sind, sondern aus der gleichen Folge von Überlagerungsbildern abgeleitet sind. Grundsätzlich ist es aber auch möglich, zwei getrennte Bildfolgen zu erzeugen, die den Untersuchung sbereich mit unterschiedlicher Projektionsgeometrie abbilden.

Eine bevorzugte Weiterbildung der Erfindung sieht vor, daß zur Darstellung des Gefäßverlaufs bei einem Patienten vor der Anfertigung der Röntgenaufnahmen eine Kontrastmittelinjektion erfolgt zur Erzeugung einer ersten Serie von Röntgenaufnahmen, die das mit Kontrastmittel gefüllte Gefäßsystem des Patienten darstellen. Dadurch ist es möglich, das Gefäßsystem darzustellen. Bei einer solchen Gefäßdarstellung läßt sich eine weitere Verbesserung dadurch erreichen, daß in geringem zeitlichen Abstand von der ersten Serie von Röntgenaufnahmen eine weitere Serie von Röntgenaufnahmen angefertigt wird, die den Patienten ohne Kontrastmittel darstellen, daß die zueinander korrespondierenden Röntgenaufnahmen der beiden Serien zur Erzeugung von Differenzbildern voneinander subtrahiert werden, und daß zur Erzeugung der Überlagerungsbilder die Differenzbilder und die synthetischen Projektionsbilder einander überlagert werden.

Ein wichtige Voraussetzung für quantitative Messungen an den Überlagerungsbildern ist, daß diese die aus den beiden bildgebenden Einrichtungen erhaltenen anatomischen Strukturen korrekt darstellen. Wenn der Röntgenbildaufnehmer der ersten bildgebenden Einrichtung jedoch ein Röntgenbildverstärker ist, können sich aufgrund der üblichen Krümmung des Eingangsschirms des Röntgenbildverstärkers sowie durch die Wirkung des Erdmagnetfeldes Verzerrungen der Röntgenaufnahmen ergeben. Diese werden in Ausgestaltung der Erfindung beseitigt durch einen ersten Korrekturschritt zur Korrektur der von dem Röntgenbildaufnehmers abhängigen Verzerrungen mit Hilfe eines ersten Satzes gespeicherter Korrekturparameter vor dem Überlagerungsschritt.

Bei einem Röntgenaufnahmesystem, bei dem ein Röntgenstrahler und der Röntgenbildaufnehmer an einem C-Bogen befestigt sind, werden die Röntgenaufnahmen auch dadurch beeinflußt, daß der C-Bogen nicht starr ist, sondern sich unter dem Einfluß der Schwerkraft und der Fliehkräfte, ggf auch durch mechanische Schwingungen verformt. Dadurch verschieben bzw. verdrehen sich die Röntgenaufnahmen gegnüber dem Idealfall (gleiche relative Lage des Röntgenstrahlers in Bezug auf den Röntgenbildaufnehmer in allen Pespektiven). Dadurch wird die Genauigkeit der Überlagerungsbilder beeinträchtigt. Diese lassen sich beseitigen durch einen zweiten Korrekturschritt zur Korrektur der durch die Änderung der relativen Position des Röntgenstrahlers in Bezug auf den Röntgenbildaufnehmer abhängigen Bildtransformation (Bildverschiebung und Bilddrehung) mit Hilfe eines zweiten Satzes gespeicherter Korrekturparameter vor dem Überlagerungsschritt.

Eine Anordnung zur Durchführung des erfindungsgemäßen Verfahrens weist eine erste bildgebende Einrichtung auf, die einen Röntgenstrahler und einen Röntgenbildaufnehmer umfaßt, die zur Anfertigung einer Serie von zweidimensionalen Röntgenaufnahmen, bei denen ein Untersuchungsobjekt aus unterschiedlichen Perspektiven auf den Röntgenbildaufnehmer projiziert wird, in Bezug auf ein Untersuchungsobjekt verstellbar sind, mit Mitteln zum Speichern der Röntgenaufnahmen und mit programmierbaren Bildverarbeitungsmitteln, die so programmiert sind, daß folgende Bildverarbeitungsoperationen durchgeführt werden:
a) Extraktion einer relevanten Struktur des Untersuchungsobjektes aus einem dreidimensionalen Bild, das von dem gleichen Untersuchungsobjekt mit einer zweiten bildgebenden Einrichtung erstellt ist
b) Für jedes Bild der Serie von zweidimensionalen Röntgenaufnahmen Berechnung eines synthetischen, zweidimensionalen Projektionsbildes der extrahierten Struktur, wobei die Struktur mit den gleichen geometrischen Parametern projiziert wird wie sie für die reale Struktur bei der Erzeugung der einzelnen Röntgenaufnahmen galten,
c) Erzeugung von Überlagerungsbildern durch Überlagerung der synthetischen Projektionsbilder mit den unter den gleichen geometrischen Parametern erstellten Röntgenaufnahmen
d) Wiedergabe der Folge der Überlagerungsbilder, so daß ein Benutzer einen quasi dreidimensionalen Eindruck von der Lage der relevanten Struktur in Bezug auf das in der Serie von zweidimensionalen Röntgenaufnahmen abgebildete Untersuchungsobjekt erhält.

Die Erfindung wird nachstehend anhand der Zeichnungen näher erläutert. Es zeigen:
Fig. 1 eine Einrichtung, mit der die Erfindung durchführbar ist, in schematischer Darstellung,
Fig. 2 den Ablauf der Bildverarbeitungsoperationen,
Fig. 3 die geometrischen Verhältnisse, die der Berechnung der Projektionsbilder zugrundeliegen und
Fig. 4 eine Folge von Überlagerungsbildern.

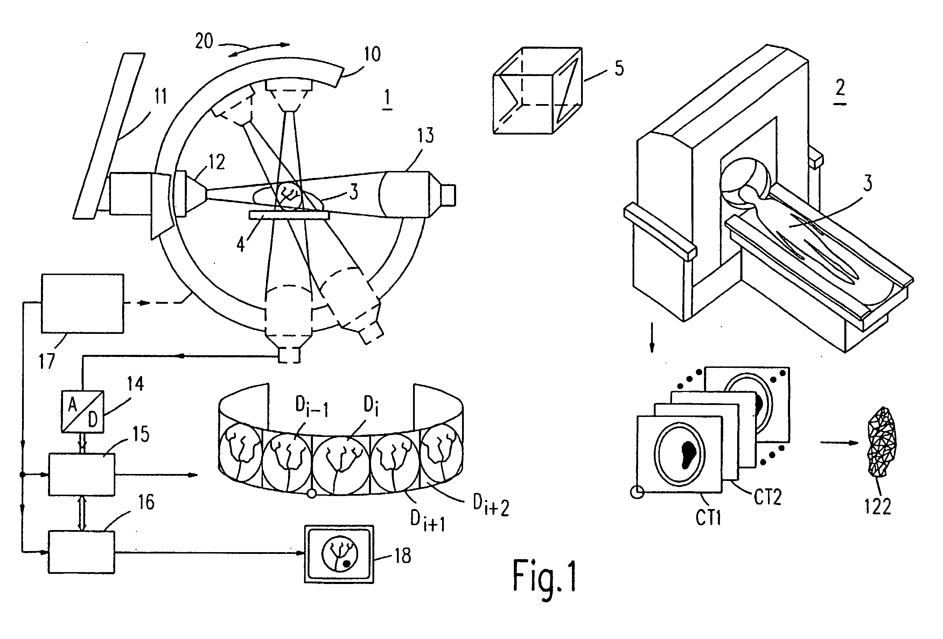

In Fig. 1 ist mit 1 eine erste bildgebende Einrichtung und mit 2 eine zweite bildgebende Einrichtung bezeichnet. Um die Bilder der ersten und der zweiten bildgebenden Einrichtung - bzw aus diesen Bildern abgeleitete Daten zueinander in Beziehung setzen zu können, wird in bekannter Weise ein Referenzrahmen 5 benutzt, der in den Bildern beider Einrichtungen mit abgebildet wird, ggf in Verbindung mit kugelförmigen Referenzmarkern. Grundsätzlich können diese Bilder aber auch anhand charkteristischer anatomischer Strukturen korreliert werden, wenn diese mit Hilfe geeigneter Bildverarbeitungsverfahren detektiert werden.

Die erste bildgebende Einrichtung dient der Erstellung zweidimensionaler Röntgenaufnahmen eines auf einem Tisch 4 befindlichen Untersuchungsobjektes 3, z.B. eines Patienten. Die zweite bildgebende Einrichtung dient zur Erzeugung eines dreidimensionalen Bildes. Als "dreidimensionales Bild" wird dabei ein Datensatz bezeichnet, der die Absorptionsverteilung in dem Untersuchungsobjekt 3 in einem dreidimensionalen Bereich wiedergibt und der aus einer Anzahl zweidimensionaler Computertomogramme CT1, CT2 ... CTm von nebeneinanderliegenden parallelen Schichten des Untersuchungsobjektes abgeleitet wird.

Die erste bildgebende Einrichtung 1 umfaßt einen kreisbogenförmigen, sogenannten C-Bogen 10 der an einem nur teilweise dargestellten Stativ 11 gehaltert ist. Dabei kann der C-Bogen einerseits um eine waagerechte Achse geschwenkt und mittels eines nicht näher dargestellten Motorantriebs in Richtung des Doppelpfeiles 20 um z.B. 180° um seinen Mittelpunkt bewegt werden. An dem C-Bogen 10 sind ein Röntgenstrahler 12 und ein Röntgenbildaufnehmer 13 befestigt, die so aufeinander ausgerichtet sind, daß von einem Untersuchungsvolumen um den erwähnten Mittelpunkt herum eine Röntgenaufnahme angefertigt werden kann. Dabei kann eine Vielzahl von Röntgenaufnahmen erzeugt werden, z.B. 100, die das Untersuchungsvolumen aus verschiedenen - reproduzierbaren - Winkelpositionen (einige sind gestrichelt angedeutet) des Bildaufnahmesystems 12, 13 abbilden.

Der Röntgenbildaufnehmer 13 kann einen Röntgenbildverstärker mit einer daran angeschlossenen Fernsehkette sein, deren Ausgangssignale von einem Analog-Digital-Wandler 14 digitalisiert und in einem Speicher 15 gespeichert werden, so daß am Ende der Untersuchung die gesamte Röntgenaufnahmeserie gespeichert ist. Diese Röntgenaufnahmen können von einer Bildverarbeitungseinheit 16 verarbeitet werden. Die erzeugten Bilder (....Dᵢ₋₁, Dᵢ, Dᵢ₊₁, Dᵢ₊₂ .....) können - einzeln oder als Bildfolge - auf einem Monitor 18 dargestellt werden. Die Steuerung der einzelnen Komponenten des bildgebenden Systems 1 erfolgt mit Hilfe einer Steuereinheit 17.

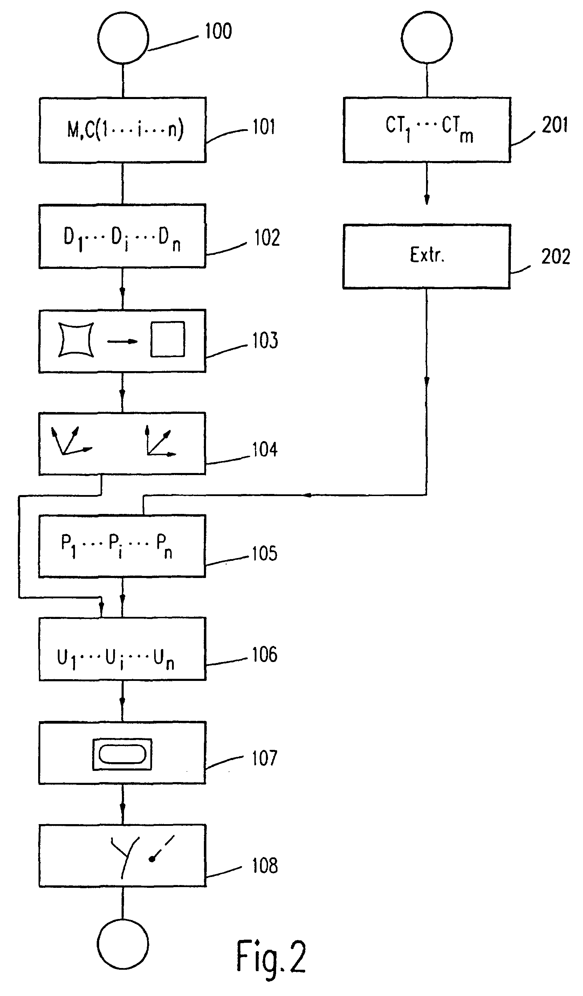

In Fig. 2 ist die Folge der Verfahrensschritte für die beiden bildgebenden Systeme dargestellt. Nach der Initialisierung (100) des ersten bildgebenden Systems wird - nach einer Kontrastmittelinjektion - eine Folge von n Röntgenaufnahmen erstellt (z.B. n=100), die das Untersuchungsobjekt und die darin befindlichen, mit Kontrastmittel gefüllten Blutgefäße darstellen (Schritt 101). Davor - oder danach - wird eine weitere Serie von Röntgenaufnahmen M erstellt, die dasselbe Objekt unter denselben Perspektiven darstellen wie die Röntgenaufnahmen C - die jedoch das Gefäßsystem nicht darstellen (weil entweder das Kontrastmittel noch nicht injiziert ist oder das Kontrastmittel sich schon so weit verteilt hat, daß es im Bild nicht mehr sichtbar wird).

Anschließend werden die Leeraufnahmen M von den korrespondierenden Kontrastbildern, die aus derselben Winkelposition aufgenommen wurden, subtrahiert (Schritt 102), so daß sich eine Folge von Differenzbildern D₁...Dᵢ...Dₙ ergibt, die für die verschiedenen Winkelpositionen nur noch das Gefäßsystem darstellen, weil die anderen anatomischen Strukturen durch die Subtraktion eliminiert werden. - Anstelle der Differenzbilder können aber auch lediglich Kontrastmittelaufnahmen (ohne Subtraktion von Leeraufnahmen) verwendet werden. In diesem Fall muß mehr Kontrastmittel injiziert werden; man erkennt dann jedoch auch noch Knochenstrukturen.

Vor oder nach der Erzeugung dieser Röntgenaufnahmen wird von derselben anatomischen Region des Patienten eine Serie von Computertomogrammen CT1...CTm erstellt, die die Absorptionsverteilung in benachbarten parallelen Ebenen der Untersuchungsregion darstellt, so daß sich ein dreidimensionales "Bild" ergibt, d.h. ein Datensatz, der die Absorptionsverteilung in einem dreidimensionalen Bereich kennzeichnet (Verfahrensschritt 201). Damit die Bilddaten, die aus den verschiedenen Modalitäten gewonnen werden, zueinander in Beziehung gesetzt werden können, wird ein Referenzrahmen gegebenenfalls in Verbindung mit Referenzmarkern benutzt, die bezüglich der Untersuchungsregion - z.B. dem Schädel des Patienten - fixiert sind und in den Röntgenaufnahmen bzw. den Computertomogrammen mit abgebildet werden. Sie können in den Röntgenaufnahmen bzw. in den Computertomogrammen mit Hilfe automatischer Bildverarbeitungsverfahren detektiert werden und als Koordinatensystem herangezogen werden, wenn es darum geht, Bilddaten der einen Modalität zu Bilddaten der anderen Modalität in Beziehung zu setzen. Dies ist im einzelnen in dem Dokument D2 beschrieben.

Im nächsten Verfahrensschritt 202 wird eine diagnostisch relevante Struktur aus den Computertomogrammen extrahiert, z.B. ein Tumor oder eine bestimmte Region im Gehirn (Ventrikel). Dies kann interaktiv durch den Benutzer erfolgen, jedoch sind auch automatische Bildverarbeitungsverfahren möglich, die diese Struktur durch Segmentierung extrahieren (202). Danach ist nicht nur Form und Größe der Struktur bekannt, sondern auch ihre Lage in Bezug auf ein mit dem Referenzrahmen bzw. den Referenzmarkern verbundenes Koordinatensystem. Im einfachsten Fall genügt es, von der Struktur auch nur ein geometrisches Attribut zu extrahieren, z.B. ihren Mittelpunkt (Schwerpunkt) oder Linien oder einfache geometrische Körper, die diese Struktur begrenzen.

Bevor diese Struktur mit den Röntgenaufnahmen in Verbindung gebracht wird, ist es in manchen Fällen noch erforderlich, die Röntgenaufnahmen zu korrigieren bzw. zu kalibrieren, um den realen Verhältnissen bei der Röntgenaufnahme Rechnung zu tragen. So können sich geometrische Verzerrungen ergeben, wenn der Röntgenbildaufnehmer einen Röntgenbildverstärker umfaßt, der einen gekrümmten Eingangsschirm aufweist und dessen Ausgangsschirmbild von dem Erdmagnetfeld beeinflußt werden kann. Zur Beseitigung dieser Verzerrungen werden im Schritt 103 die Differenzbilder D₁...Dₙ einer geometrischen Transformation unterzogen, deren Parameter in einem vorangehenden Kalibrierverfahren ermittelt und gespeichert sind, bei dem vorzugsweise ein regelmäßiges Gitter im Strahlengang angeordnet und seine Abbildung in einer Röntgenaufnahme ausgewertet wird. Im einzelnen ist dies in dem Dokument D1 beschrieben. Wenn der Röntgenbildaufnehmer keine solchen geometrischen Verzerrungen aufweist, kann dieser Verfahrensschritt entfallen.

Weitere Faktoren, die die Genauigkeit des erfindungsgemäßen Verfahrens beeinträchtigen können, ergeben sich daraus, daß der C-Bogen nicht absolut starr ist. Er verformt sich vielmehr unter dem Einfluß der Schwerkraft und der Fliehkräfte, so daß sich der Abstand des Röntgenstrahlers von dem Bildverstärker je nach der Lage des C-Bogens im Raum ändern kann. Weiterhin kann diese Verformung zur Folge haben, daß sich das Isozentrum (welches sich auf dem den Röntgenstrahler mit dem Mittelpunkt des Bildaufnehmers verbindenden Zentralstrahl befindet), mit dem das für die Röntgenaufnahme relevante Koordinatensystem verknüpft ist, sich von Röntgenaufnahme zu Röntgenaufnahme verschiebt und dreht. Die dadurch bewirkten Veränderungen der Röntgenaufnahmen stören normalerweise nicht, solange man die Röntgenaufnahmen für sich allein betrachtet. Wenn man aber Bilddaten aus verschiedenen Röntgenaufnahmen miteinander oder mit Bilddaten aus dem CT-Bild in Beziehung setzen will, wird dadurch die erzielbare Genauigkeit beeinträchtigt.

Die im Verfahrensschritt 104 durchgeführte Korrektur der dadurch bewirkten Effekte basiert auf der Tatsache, daß sich das System 10, 11, 12, 13 (vergl. Fig. 1) bei einer Rotation in Richtung des Doppelpfeiles 20 in reproduzierbarer Weise verformt. Die Verformung kann in einem vorangehenden Kalibrierverfahren mit Hilfe geeigneter Kalibrierkörper ermittelt werden, und die daraus - für jede einzelne Winkelposition - ableitbaren Korrekturparameter werden zur Korrektur der in diesen Winkelpositionen erzeugten Röntgenaufnahmen herangezogen. Auch dieses Kalibrier- und Korrekturverfahren ist im einzelnen in dem Dokument D1 beschrieben. Es kann entfallen, wenn der C-Bogen so starr ist, daß sich die Verformungen nicht auf die Röntgenaufnahmen auswirken können.

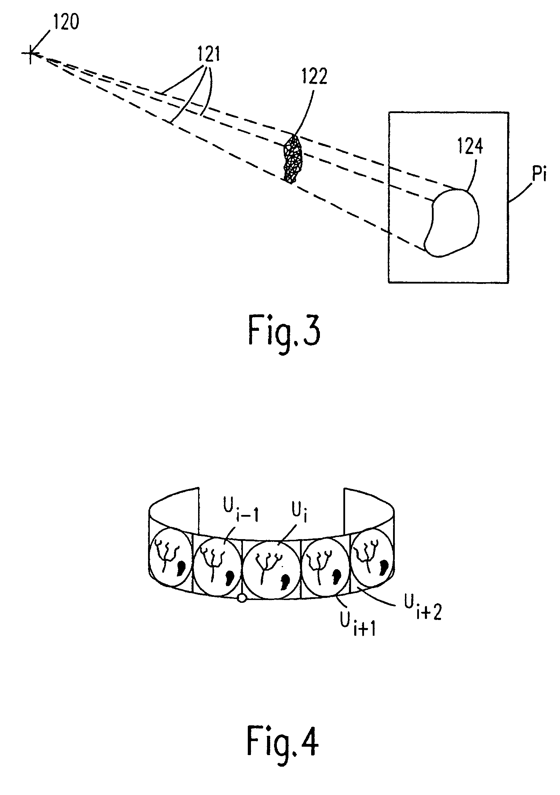

Im nächsten Verfahrensschritt 105 werden Projektionsbilder der extrahierten Struktur erzeugt, und zwar eines für jedes Differenzbild D₁...Dᵢ...Dₙ. Fig. 3 stellt dies für ein einziges Projektionsbild dar, wobei das der (zu dem Röntgenstrahler 12 korrespondierende) Projektionszentrum mit 120, die davon ausgehenden Projektionstrahlen mit 121 und die extrahierte Struktur mit 122 bezeichnet sind. Das (in seiner Lage mit dem Röntgenbildaufnehmer 13 korrespondierende) Projektionsbild ist mit Pᵢ bezeichnet. Die Berechnung eines solchen Projektionsbildes kann so erfolgen, daß ermittelt wird, ob sich auf einem zu einem Bildpunkt im Projektionsbild Pᵢ führenden Projektionstrahl 121 mindestens ein Volumenelement (voxel) der extrahierten Struktur befindet. Ist dies der Fall, wird dem Bildpunkt ein geigneter Bildwert zugeordnet; wenn nicht der Bildwert 0. Dies wird für alle Bildpunkte wiederholt, sodaß sich ein Projektionsbild Pᵢ ergibt, das eine Projektion 124 der Struktur darstellt. Dies wird für alle Winkelpositionen des System 12-13 wiederholt, in denen Röntgenaufnahmen bzw die daraus abgeleiteten Differenzbilder erzeugt wurden.

Jedes auf diese Weise erzeugte Projektionsbild Pᵢ ist einer Röntgenaufnahme bzw. einem Differenzbild Dᵢ zugeordnet, wobei die Lage des Projektionszentrums 120 und des Projektionsbildes P; in bezug auf die extrahierte Struktur 122 von der Lage des Röntgenstrahlers bzw. des Röntgenbildaufnehmers in bezug auf die reale Struktur bei der Anfertigung der korrespondierenden Röntgenaufnahme bestimmt sind.

Die auf diese Weise erzeugten synthetischen Projektionsbilder können die extrahierte Struktur mit einem beliebig vorgebbaren Kontrast darstellen, aber auch farbig. Es kommt ja lediglich darauf an, die anatomischen Details aus den verschiedenen Bildern geometrisch zutreffend wiederzugeben, aber nicht hinsichtlich des Kontrastes. - Auf diese Weise wird im Projektionsschritt 105 für jede Röntgenaufnahme ein synthetisches Projektionsbild erzeugt, bei dem die projizierte Struktur im Projektionsbild die gleiche Form und Lage hat, wie sie die reale Struktur in den - gegebenenfalls korrigierten - Differenzbildern D₁...Dᵢ...Dₙ haben würde, wenn sie dort abgebildet werden könnte.

Im nächsten Verfahrensschritt 106 werden die Differenzbilder D₁...Dᵢ...Dₙ, die im wesentlichen das Gefäßsystem abbilden, und die synthetischen Projektionsbilder P₁...Pᵢ...Pₙ, die die aus dem CT-Bild extrahierte Struktur darstellen, einander überlagert, so daß eine Folge von Überlagerungsbildern U₁...Uᵢ,..Uₙ (Fig. 4) entsteht, die beide anatomische Strukturen in geometrisch korrekter Zuordnung wiedergibt. Diese Bildfolge kann im darauf folgenden Verfahrensschritt 107 auf dem Monitor wiedergegeben werden, so daß ein quasi dreidimensionaler Bildeindruck entsteht, der eine Behandlungsplanung, z.B. die Vorgabe eines Biopsiepfades, zur Einführung eines Biopsienadel in die Struktur - oder die Beurteilung eines zuvor berechneten Biopsiepfades wesentlich erleichtert. Mit einem dreidimensionalen Ziehkreuz (Cursor) kann der Biopsiepfad interaktiv festgelegt werden. Abstandsmessungen sind mit Hilfe eines statischen Bildwiedergabe (Verfahrensschritt 108) möglich, wobei jeweils zwei Überlagerungsbilder (mit unterschiedlichem Projektionswinkel dargestellt werden. Es können auch Volumenmessungen durchgeführt werden. Danach ist das Verfahren beendet.

Vorstehend wurden zunächst die synthetischen Projektionsbilder 105 berechnet und gespeichert, und erst danach wurden die einzelnen Überlagerungsbilder erzeugt. Es ist jedoch auch möglich, während der Wiedergabe einer Röntgenaufnahme bzw. eines Differenzbildes das zugehörige synthetische Projektionsbild zu berechnen und das Differenzbild und das gerade berechnete Projektionsbild auf dem Monitor 18 zu überlagern, und zwar auch dann, wenn die Röntgenaufnahmen bzw. die daraus abgeleiteten Differenzbilder relativ schnell nacheinander wiedergegeben werden. Es ist dann also nicht erforderlich, vorab sämtliche synthetischen Projektionsbilder zu berechnen und sie erst danach den Differenzbildern bzw. Röntgenaufnahmen zu überlagern.

Es ist auch eine stereoskopische Betrachtung möglich, bei der jeweils gleichzeitig ein Stereobildpaar wiedergegeben wird, das mit den üblichen Mitteln betrachtet werden kann. Dazu müssen keine gesonderten Röntgenaufnahmen erzeugt werden. Es genügt vielmehr, jeweils zwei Überlagerungsbilder wiederzugeben, die die Untersuchungsregion aus zwei Winkelpositionen wiedergeben, die sich um etwa 6° unterscheiden - z.B. die Überlagerungsbilder Uᵢ₋₁ und Uᵢ₊₁ oder die Bilder Uᵢ und Uᵢ₊₂ (vergl. Fig. 4). Diese stereoskopische Darstellung erleichtert nicht nur die Planung eines Biopsiepfades sondern auch die stereotaktische Messungen in den Überlagerungsbildern.

## Patentansprüche

1. Röntgenaufnahme-Verfahren, bei dem mit einer ersten bildgebenden Einrichtung (1) eine Serie von zweidimensionalen Röntgenaufnahmen (D₁...Dᵢ...Dₙ) angefertigt und digital gespeichert wird, bei denen ein Untersuchungsobjekt (3, 5) aus unterschiedlichen Perspektiven auf einen Röntgenbildaufnehmer projiziert wird, und das folgende Verfahrensschritte umfaßt:
a) Erstellung eines dreidimensionalen Bildes (CT1...CTm) von dem gleichen Untersuchungsobjekt mit einer zweiten bildgebenden Einrichtung (2),
b) Extraktion einer relevanten Struktur (122) des Untersuchungsobjektes aus dem dreidimensionalen Bild,
c) Für jedes Bild der Serie von zweidimensionalen Röntgenaufnahmen, Berechnung eines synthetischen, zweidimensionalen Projektionsbildes (Pᵢ) der extrahierten Struktur, wobei die Struktur mit den gleichen geometrischen Parametern projiziert wird wie sie für die reale Struktur bei der Erzeugung der einzelnen Röntgenaufnahmen galten,
d) Erzeugung von Überlagerungsbildern (U₁...Uᵢ...Uₙ) durch Überlagerung der synthetischen Projektionsbilder (P₁...Pᵢ...Pₙ) und der mit den gleichen geometrischen Parametern erstellten Röntgenaufnahmen (D₁...Dᵢ...Dₙ),
e) Wiedergabe der Folge der Überlagerungsbilder (U₁...Uᵢ...Uₙ), so daß ein Benutzer einen quasi dreidimensionalen Eindruck von der Lage der relevanten Struktur in Bezug auf das in der Serie von zweidimensionalen Röntgenaufnahmen abgebildete Untersuchungsobjekt erhält.

2. Verfahren nach Anspruch 1,
**dadurch gekennzeichnet, daß** als zweite bildgebende Einrichtung ein Röntgen-Computertomograph (2) verwendet wird, wobei zur Erstellung eines dreidimensionalen Bildes von dem Untersuchungsobjekt (3) eine Anzahl von Computer-Tomogrammen (CT1.....CTm) von parallelen Schichten erstellt wird.

3. Verfahren nach Anspruch 1,
**dadurch gekennzeichnet, daß** zur Erzeugung einer Folge von Stereo-Bildpaaren zwei Bild-Folgen wiedergegeben werden, die beide aus der einen Folge der Überlagerungsbilder (U₁...Uᵢ...Uₙ) abgeleitet werden, wobei die beiden Bildfolgen um einige Überlagerungsbilder gegeneinander versetzt sind.

4. Verfahren nach Anspruch 1,
**dadurch gekennzeichnet, daß** das Untersuchungsobjekt ein Patient ist, und daß zur Darstellung des Gefäßverlaufs bei dem Patienten vor der Anfertigung der Röntgenaufnahmen eine Kontrastmittelinjektion erfolgt zur Erzeugung einer ersten Serie von Röntgenaufnahmen, die das mit Kontrastmittel gefüllte Gefäßsystem des Patienten darstellen.

5. Verfahren nach Anspruch 4,
**dadurch gekennzeichnet, daß** in geringem zeitlichen Abstand von der ersten Serie von Röntgenaufnahmen eine weitere Serie von Röntgenaufnahmen angefertigt wird, die den Patienten ohne Kontrastmittel darstellen, daß die zueinander korrespondierenden Röntgenaufnahmen der beiden Serien zur Erzeugung von Differenzbildern voneinander subtrahiert werden (102), und daß zur Erzeugung der Überlagerungsbilder die Differenzbilder und die synthetischen Projektionsbilder einander überlagert werden (106).

6. Verfahren nach Anspruch 1,
**gekennzeichnet durch** einen ersten Korrekturschritt (103) zur Korrektur der von dem Röntgenbildaufnehmer (13) abhängigen Verzerrungen mit Hilfe eines ersten Satzes gespeicherter Korrekturparameter vor dem Überlagerungsschritt (106).

7. Verfahren nach Anspruch 1,
**gekennzeichnet durch** einen zweiten Korrekturschritt (104) zur Korrektur der **durch** die Änderung der relativen Position des Röntgenstrahlers (12) in Bezug auf den Röntgenbildaufnehmer (13) abhängigen Bildtransformationen mit Hilfe eines zweiten Satzes gespeicherter Korrekturparameter vor dem Überlagerungsschritt (106).

8. Anordnung zur Durchführung des Verfahrens nach Anspruch 1, mit einer ersten bildgebenden Einrichtung (1) die einen Röntgenstrahler (12) und einen Röntgenbildaufnehmer (13) umfaßt, die zur Anfertigung einer Serie von zweidimensionalen Röntgenaufnahmen (D₁...Dᵢ...Dₙ), bei denen ein Untersuchungsobjekt (3,5) aus unterschiedlichen Perspektiven auf den Röntgenbildaufnehmer projiziert wird, in Bezug auf ein Untersuchungsobjekt verstellbar sind, mit Mitteln (15) zum Speichern der Röntgenaufnahmen und mit programmierbaren Bildverarbeitungsmitteln (16), die so programmiert sind, daß folgende Bildverarbeitungsoperationen durchgeführt werden:
a) Extraktion einer relevanten Struktur (122) des Untersuchungsobjektes (3) aus einem dreidimensionalen Bild (CT1.......CTm), das von dem gleichen Untersuchungsobjekt mit einer zweiten bildgebenden Einrichtung (2) erstellt ist
b) Für jedes Bild der Serie von zweidimensionalen Röntgenaufnahmen Berechnung eines synthetischern zweidimensionalen Projektionsbildes (Pᵢ) der extrahierten Struktur, wobei die Struktur mit den gleichen geometrischen Parametern projiziert wird wie sie für die reale Struktur bei der Erzeugung der einzelnen Röntgenaufnahmen galten
c) Erzeugung von Überlagerungsbildern (U₁...Uᵢ...Uₙ) durch Überlagerung der synthetischen Projektionsbilder (P₁...Pᵢ...Pₙ) mit den unter den gleichen geometrischen Parametern erstellten Röntgenaufnahmen (D₁...Dᵢ...Dₙ)
d) Wiedergabe der Folge der Überlagerungsbilder (U₁...Uᵢ...Uₙ), so daß ein Benutzer einen quasi dreidimensionalen Eindruck von der Lage der relevanter Struktur in Bezug auf das in der Serie von zweidimensionalen Röntgenaufnahmen abgebildete Untersuchungsobjekt erhält.

9. Anordnung nach Anspruch 8,
**dadurch gekennzeichnet, daß** sie einen C-Bogen (10) umfaßt, an dem der Röntgenstrahler (12) und der Röntgenbildaufnehmer (13) befestigt sind, und daß der C-Bogen auf einer Kreisbahn in eine Vielzahl von Aufnahme-Positionen bewegbar ist.

## Claims

1. An X-ray imaging method in which a series of two-dimensional X-ray images (D₁ ... Dᵢ ... Dₙ) is formed and digitally stored by means of a first imaging device (1) and in which an object to be examined (3, 5) is projected onto an X-ray image pick-up device from different perspectives, which method includes the following steps:
a) forming a three-dimensional image (CT₁ ... CTₘ) of the same object to be examined by means of a second imaging device (2),
b) extracting a relevant structure (122) of the object to be examined from the three-dimensional image,
c) calculating, for each image of the series of two-dimensional X-ray images, a synthetic, two-dimensional projection image (Pᵢ) of the extracted structure, the structure being projected with the same geometrical parameters as used for the real structure during the formation of the individual X-ray images,
d) forming superposition images (U₁ ... Uᵢ ... Uₙ) by superposing the synthetic projection images (P₁ ... Pᵢ ... Pₙ) and the X-ray images (D₁ ... Dᵢ ... Dₙ) formed with the same parameters,
e) displaying the series of superposition images (U₁ ... Uᵢ ... Uₙ) in such a manner that a user is given a quasi three-dimensional impression of the position of the relevant structure in relation to the object to be examined and depicted by the series of two-dimensional X-ray images.

2. A method as claimed in Claim 1, **characterized in that** an X-ray computed tomography apparatus (2) is used as the second imaging device, which apparatus forms a number of computed tomograms (CT₁ ... CTₘ) of parallel slices so as to form a three-dimensional image of the object (3) to be examined.

3. A method as claimed in Claim 1, **characterized in that** two series of images are displayed in order to form a series of stereo image pairs, both said two series being derived from the one series of superposition images (U₁ ... Uᵢ ... Uₙ) and being offset a few superposition images relative to one another.

4. A method as claimed in Claim 1, **characterized in that** the object to be examined is a patient and that the imaging of the vascular system of the patient involves the injection of a contrast medium, prior to the formation of the X-ray images, in order to form a first series of X-ray images which depict the vascular system of the patient filled with contrast medium.

5. A method as claimed in Claim 4, **characterized in that** a further series of X-ray images is formed at a small distance in time from the first series of X-ray images, which further series depicts the patient without contrast medium, the corresponding X-ray images of the two series being subtracted (102) from one another in order to form difference images, the difference images and the synthetic projection images being superposed (106) so as to form the superposition images.

6. A method as claimed in Claim 1, **characterized in that** it includes a first correction step (103) for the correction of the distortions due to the X-ray image pick-up device (13), which correction step applies a first set of stored correction parameters prior to the superposition step (106).

7. A method as claimed in Claim 1, **characterized in that** it includes a second correction step (104) for the correction of the image transformations which are due to the changing of the relative position of the X-ray source (12) with respect to the X-ray image pick-up device (13), which second correction step applies a second set of stored correction parameters prior to the superposition step (106).

8. A device for carrying out the method claimed in Claim 1, including a first imaging device (1) with an X-ray source (12) and an X-ray image pick-up device (13) which are adjustable in relation to an object (3,5) to be examined in order to form a series of two-dimensional X-ray images (D₁ ... Dᵢ ... Dₙ), where the object to be examined is projected onto the image pick-up device from different perspectives, and also including means (15) for storing the X-ray images, and programmable image processing means (16) which are programmed so that the following image processing operations are performed:
a) extracting a relevant structure (122) of the object to be examined (3) from a three-dimensional image (CT₁ ... CTₘ) of the same object to be examined which has been formed by a second imaging device (2),
b) calculating, for each image of the series of two-dimensional X-ray images, a synthetic two-dimensional projection image (Pᵢ) of the extracted structure, the structure being projected with the same geometrical parameters as used for the real structure during the formation of the individual X-ray images,
c) forming superposition images (U₁ ... Uᵢ ... Uₙ) by superposing the synthetic projection images (P₁ ... Pᵢ ... Pₙ) and the X-ray images (D₁ ... Dᵢ ... Dₙ) formed with the same parameters,
d) displaying the series of superposition images, (U₁ ... Uᵢ ... Uₙ) in such a manner that a user is given a quasi three-dimensional impression of the position of the relevant structure in relation to the object to be examined and depicted by the series of two-dimensional X-ray images.

9. A device as claimed in Claim 8, **characterized in that** it includes a C-arm (10) whereto the X-ray source (12) and the X-ray image pick-up device (13) are attached, and that the C-arm can be moved to a plurality of exposure positions along a circular path.

## Revendications

1. Procédé radiographique, une série de clichés radiographiques bidimensionnels (D₁...Dᵢ...Dₙ) étant tirée et enregistrée numériquement par un premier dispositif d'imagerie (1), un objet d'examen (3, 5) étant projeté à partir de différentes perspectives sur un appareil radiographique et qui comprend les étapes de procédé suivantes :
a) Elaboration d'une image tridimensionnelle (CT₁...CTₘ) du même objet d'examen avec un deuxième dispositif d'imagerie (2) ;
b) extraction d'une structure pertinente (122) de l'objet d'examen à partir de l'image tridimensionnelle ;
c) pour chaque image de la série de clichés radiographiques bidimensionnels, calcul d'une image de projection bidimensionnelle synthétique (Pᵢ) de la structure extraite, la structure étant projetée avec les mêmes paramètres géométriques que pour la structure réelle pour la production des différents clichés radiographiques individuels.
d) production d'images de superposition U₁ ... Uᵢ...Un par superposition des images de projection synthétiques (P₁...Pᵢ...Pₙ) et des clichés radiographiques tirés avec les mêmes paramètres géographiques (D₁ ...Dᵢ...Dₙ)
e) Reproduction de la séquence des images de superposition (U₁...Uᵢ...Uₙ) de telle sorte qu'un utilisateur obtienne une impression quasi tridimensionnelle de la position de la structure pertinente par rapport à l'objet d'examen représenté dans la série des clichés radiographiques bidimensionnels.

2. Procédé selon la revendication 1,
**caractérisé en ce qu'**un tomographe informatisé (2) est utilisé comme deuxième dispositif d'imagerie, plusieurs tomogrammes informatisés (CT₁ ... CTₘ) de couches parallèles étant tirés pour la production d'une image tridimensionnelle de l'objet d'examen 3.

3. Procédé selon la revendication 1,
**caractérisé en ce que** deux séquences d'images sont reproduites pour la production d'une séquence de paires d'images stéréo, les deux étant dérivés d'une des séquences des images de superposition (U1 ... Uᵢ...Uₙ), deux séquences étant décalées l'une par rapport à l'autre de quelques images de superposition.

4. Procédé selon la revendication 1,
**caractérisé en ce que** l'objet de l'examen est un patient et qu'un produit de contraste est injecté pour la représentation du système vasculaire du patient avant la production des clichés radiographiques en vue de la production d'une première série de clichés radiographiques qui représentent le système vasculaire du patient rempli de produit de contraste.

5. Procédé selon la revendication 4,
**caractérisé en ce qu'**une nouvelle série de clichés radiographiques est tirée à un intervalle temporel minime de la première série de clichés radiographiques et représente le patient sans produit de contraste de telle sorte que les clichés radiographiques correspondants des deux séries soient soustraits l'un de l'autre pour la production d'images différentielles (102) et que les images différentielles et les images de projection synthétiques sont superposées l'une à l'autre pour la production d'images de superposition (106).

6. Procédé selon la revendication 1,
**caractérisé par** une première étape de correction (103) pour la correction des distorsions dépendant du capteur d'images radiographiques (13) à l'aide d'un premier jeu de paramètres de correction enregistrés avec l'étape de superposition (106).

7. Procédé selon la revendication 1,
**caractérisé par** une deuxième étape de correction (104) pour la correction des transformations d'image dépendant de la variation de la position relative du générateur de rayons X (12) par rapport au capteur d'images radiographiques (13) à l'aide d'un deuxième jeu de paramètres de correction enregistrés avant l'étape de superposition (106).

8. Dispositif d'exécution du procédé selon la revendication 1 avec un premier dispositif d'imagerie (1) qui comprend un générateur de rayons X (12) et un appareil radiographique (13) qui sont déplaçables par rapport à un objet d'examen pour la production d'une série de clichés radiographiques bidimensionnels (D₁ ...Dᵢ ...Dₙ), un objet d'examen (3, 5) étant projeté à partir de différentes perspectives sur l'appareil radiographique, avec des moyens (15) pour l'enregistrement des clichés radiographiques et avec des moyens de traitement d'images programmables (16) programmés de telle sorte que les opérations suivantes de traitement de l'image soient effectuées:
a) extraction d'une structure pertinente (122) de l'objet d'examen (3) à partir de l'image tridimensionnelle (CT₁ ... CTₘ) qui est créée du même objet d'examen avec un deuxième dispositif d'imagerie (2);
b) pour chaque image de la série de clichés radiographiques bidimensionnels, calcul d'une image de projection bidimensionnelle synthétique (Pᵢ) de la structure extraite, la structure étant projetée avec les mêmes paramètres géométriques que pour la structure réelle pour la production des différents clichés radiographiques individuels.
c) production d'images de superposition (U₁ ... Uᵢ...Uₙ) par superposition des images de projection synthétiques (P₁...Pᵢ...Pₙ) et des clichés radiographiques tirés avec les mêmes paramètres géographiques (D₁ ...Dᵢ ...Dₙ)
d) Reproduction de la séquence des images de superposition (U₁...Uᵢ...Uₙ) de telle sorte qu'un utilisateur obtienne une impression quasi tridimensionnelle de la position de la structure pertinente par rapport à l'objet d'examen représenté dans la série des clichés radiographiques bidimensionnels.

9. Dispositif selon la revendication 8,
**caractérisé en ce qu'**il comprend un support arqué en C (10) sur lequel sont fixés le générateur de rayons X (12) et l'appareil radiographique (13) et que le support arqué en C est mobile sur une trajectoire circulaire dans une multitude de positions d'enregistrement.
